# EUROPÄISCHE PATENTSCHRIFT

(11) **EP 0 597 329 B1**
(45) Veröffentlichungstag und Bekanntmachung des Hinweises auf die Patenterteilung: **05.02.1997**
(21) Anmeldenummer: 93117478.3
(22) Anmeldetag: 28.10.1993
(51) Int. Cl.: C07C 69/63, C07C 67/307, C07C 49/167, C07C 45/63, C07C 235/84, C07C 231/12, C07C 69/716

(54) **Verfahren zur Herstellung von alpha-Fluor -beta-dicarbonylverbindungen**
Process for the preparation of alpha-fluor-beta dicarbonyle compounds
Procédé de préparation de composés alpha-fluorés-bêta-dicarbonylés

(30) Priorität: 10.11.1992 DE 4237882
(43) Veröffentlichungstag der Anmeldung: 18.05.1994
(73) Patentinhaber: BAYER AG, 51368 Leverkusen (DE)
(72) Erfinder: Böhm, Stefan, Dr., D-51065 Köln (DE); Marhold, Albrecht, Dr., D-51373 Leverkusen (DE)

(56) Entgegenhaltungen:
- EP-A- 0 005 810

## Beschreibung

Die vorliegende Erfindung betrifft ein vorteilhaftes und auch in technischem Maßstab einfach durchzuführendes Verfahren zur Herstellung von α-Fluor-β-dicarbonylverbindungen.

Es ist bekannt, daß man Ethyl-bromfluoracetat mit tri-n-Butylphosphin zum entsprechenden Phosphoniumsalz umsetzen, dieses durch Reaktion mit n-Butyllithium bei -78°C in das entsprechende Ylen überführen und daraus durch Acylierung und Verseifung α-Fluor-β-keto-ester erhalten kann (s. J. Org. Chem. 56, 273-277 (1991)). Nachteilig ist dabei die schwierige Zugänglichkeit der Ausgangsprodukte, die Mehrstufigkeit des Verfahrens und die notwendige komplizierte Arbeitsweise z.B. bei der Handhabung von Phosphinen und n-Butyllithium, die besonderen sicherheitstechnischen Aufwand und tiefe Temperaturen erfordern.

Gemäß einem anderen bekannten Verfahren kann man α-Fluor-β-dicarbonylverbindungen herstellen, indem man Fluordichlormethan mit Dioxen umsetzt und das Reaktionsprodukt einer sauer katalysierten Alkoholyse unterwirft (s. J. Org. Chem. 54, 5618-5620 (1989)). Nachteilig ist die schwierige Handhabung der toxischen Stoffe Fluordichlormethan und Dioxen und die für die Verseifung benötigte Reaktionszeit von einer Woche.

Schließlich ist auch noch bekannt, daß man 1,3-Dicarbonylverbindungen durch Umsetzung mit N-Fluor-perfluoralkylsulfonimiden regioselektiv in der α-Position fluorieren kann (s. J. Chem. Soc. Chem Comm. 1991, 179). Bei diesem Verfahren ist die schwierige Zugänglichkeit des benötigten Sulfonimids nachteilig.

Es gibt bisher also noch kein auf einfache Weise und im technischen Maßstab durchführbares Verfahren zur Herstellung von α-Fluor-β-dicarbonylverbindungen.

Es wurde nun ein Verfahren zur Herstellung von α-Fluor-β-dicarbonylverbindungen der Formel (I) gefunden, 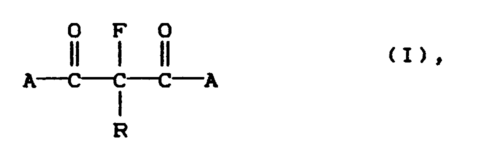
in der
die beiden Reste A gleich oder verschieden sein können und jeweils für Alkyl, Aryl, Alkoxy, Aryloxy oder eine Aminogruppe und
R für Wasserstoff, Fluor, Alkyl oder Aryl stehen,
das dadurch gekennzeichnet ist, daß man eine Dicarbonylverbindung der Formel (II) 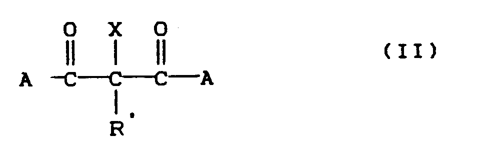
in der
- X: für Chlor, Brom oder Iod steht,
- A: die bei Formel (I) angegebene Bedeutung hat und
- R': die bei Formel (I) für R angegebene Bedeutung hat und zusätzlich nach für Chlor, Brom oder Jod stehen kann,
bei Temperaturen von 20°C bis 100°C mit einem Anlagerungsprodukt von Fluorwasserstoff an ein Trialkylamin umsetzt.

Falls im Einsatzprodukt der Formel (II) R' für Chlor, Brom oder Jod steht wird eine α,α-Difluor-β-dicarbonylverbindung erhalten, daß heißt eine Verbindung der Formel (I), in der R für Fluor steht.

In den Formeln (I) und (II) kann A beispielsweise für geradkettiges oder verzweigtes, unsubstituiertes oder substituiertes Alkyl, unsubstituiertes oder substituiertes Aryl, geradkettiges oder verzweigtes, unsubstituiertes oder substituiertes Alkoxy, unsubstituiertes oder substituiertes Aryloxy oder eine unsubstituierte oder substituierte Aminogruppe der Formeln (III) bis (V) stehen

NH₂ , (III)

NHR¹ (IV)

und

NR²R³ (V),

in denen

R¹, R² und R³ Alkyl, vorzugsweise C₁-C₆-Alkyl, oder Aryl, vorzugsweise Phenyl, bedeuten. R² und R³ können dabei gleich oder verschieden sein.

Bei den ggf. in den Alkyl- und Alkoxygruppen vorhandenen Substituenten kann es sich beispielsweise um Halogenatome, vorzugsweise Fluor, Chlor und/oder Brom oder Nitrogruppen handeln.

Bei den ggf. an Aryl- und Aryloxygruppen vorhandenen Substituenten kann es sich beispielsweise um C₁-C₆-Alkylgruppen, vorzugsweise Methyl oder Ethyl, Halogenatome, vorzugsweise Fluor, Chlor und/oder Brom, oder Nitrogruppen handeln.

In der Bedeutung von Alkyl und Alkoxy enthält A vorzugsweise 1 bis 6 C-Atome, insbesondere 1 bis 2 C-Atome, und in der Bedeutung von Aryl und Aryloxy steht A vorzugsweise für Phenyl.

In den Formeln (I) und (II) können R und R' beispielsweise für Wasserstoff, geradkettiges oder verzweigtes, unsubstituiertes oder substituiertes C₁-C₁₂-Alkyl oder unsubstituiertes oder substituiertes Phenyl stehen. Als Substituenten für Alkylgruppen kommen beispielsweise Halogenatome oder Nitrogruppen infrage, als Substituenten für Arylgruppen beispielsweise C₁-C₆-Alkylgruppen, Halogenatome oder Nitrogruppen. In Formel (II) kann R' zusätzlich für Chlor, Brom oder Jod, insbesondere für Chlor oder Brom, stehen.

Vorzugsweise stehen R und R' für Wasserstoff.

In Formel (II) steht X vorzugsweise für Chlor oder Brom.

Einige ausgewählte Beispiele für einzusetzende Dicarbonylverbindungen der Formel (II) sind:

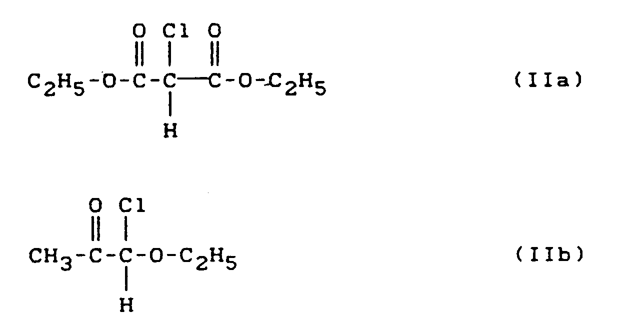

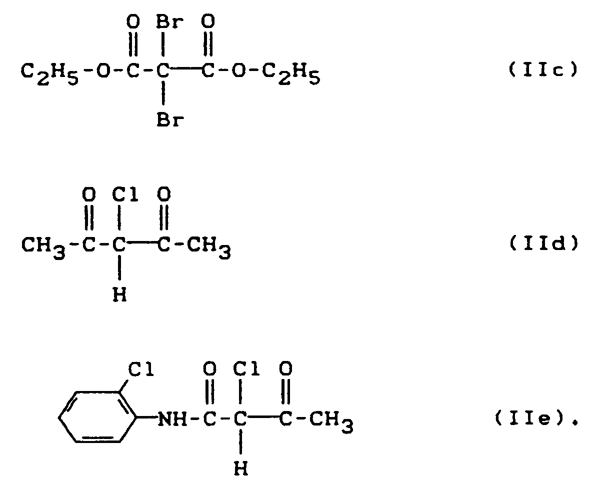

Einige ausgewählte Beispiele für erfindungsgemäß herstellbare α-Fluor-β-dicarbonylverbindungen der Formel (I) sind:

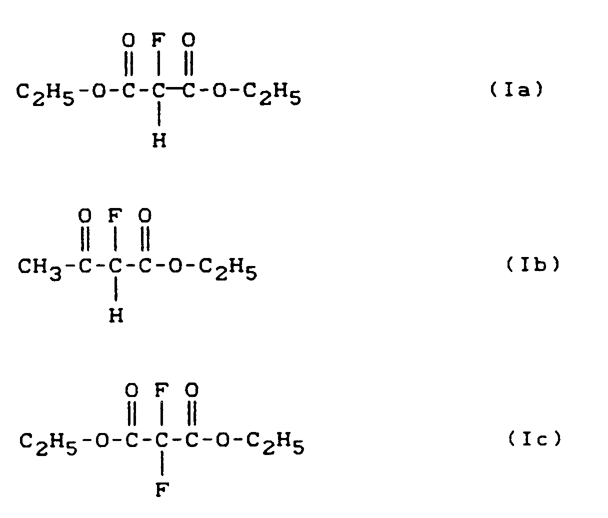

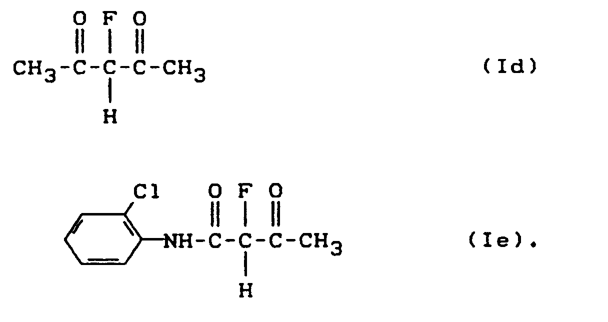

Bevorzugte Reaktionstemperaturen für das erfindungsgemäße Verfahren sind solche im Bereich 50 bis 90°C.

Bei den Anlagerungsprodukten von Fluorwasserstoff an Trialkylamine kann es sich beispielsweise um solche handeln, die pro Mol Trialkylamin 1 bis 2,8 Mole Fluorwasserstoff enthalten. Vorzugsweise liegt dieses Verhaltnis bei 1:1,5 bis 2,5, besonders bevorzugt bei 1:1,8 bis 2,2.

Häufig sind Anlagerungsprodukte von 3 Molen Fluorwasserstoff an 1 Mol Trialkylamin gut zugänglich. Man kann daraus Anlagerungsprodukte mit geringerem Gehalt an Fluorwasserstoff herstellen, auch in situ, indem man freies Trialkylamin in der entsprechenden Menge zufügt.

Als Trialkylamine kommen beispielsweise solche in Frage, die gleiche oder verschiedene Alkylgruppen mit je 1 bis 6 C-Atomen enthalten. Vorzugsweise enthalten sie drei gleiche Alkylgruppen. Besonders bevorzugt ist Triethylamin.

Anlagerungsprodukte von Fluorwasserstoff an Trialkylamin kann man, bezogen auf Dicarbonylverbindungen der Formel (II), beispielsweise in Mengen von 1 bis 4 Molen, einsetzen. Bevorzugt setzt man 1 bis 3 Mole des Anlagerungsproduktes auf 1 Mol Dicarbonylverbindung der Formel (II) ein.

Man kann das erfindungsgemäße Verfahren in Anwesenheit oder Abwesenheit eines Lösungsmittels durchführen. Als Lösungsmittel kommen beispielsweise Nitrile, insbesondere Acetonitril, Dialkylamide, insbesondere Dimethylformamid und chlorierte Kohlenwasserstoffe, insbesondere Methylenchlorid in Frage.

Die Aufarbeitung des nach der Reaktion vorliegenden Gemisches kann beispielsweise so erfolgen, daß man zunächst evtl. vorhandenes Lösungsmittel im Vakuum abzieht, dann das Gemisch auf Wasser austrägt, mit einem mit Wasser nicht mischbaren organischen Lösungsmittel extrahiert, z.B. mit Methylenchlorid und abschließend die hergestellte α-Fluor-β-dicarbonylverbindung der Formel (I) durch Fraktionierung aus der organischen Phase isoliert.

Das erfindungsgemäße Verfahren zeichnet sich durch eine Reihe von Vorteilen aus. Für es werden nur gut zugängliche, meist im Handel erhältliche Ausgangsmaterialien und Hilfsmittel benötigt, α-Fluor-β-dicarbonylverbindungen der Formel (I) werden auf einfache Weise und in guten Ausbeuten erhalten und besonderer Aufwand für die Handhabung besonders toxischer Substanzen ist nicht notwendig.

α-Fluor-β-dicarbonylverbindungen der Formel (I) sind wichtige Zwischenprodukte, beispielsweise zur Herstellung von α-Fluoracrylsäureestern nach dem in der EP-OS 203 462 beschriebenen Verfahren. Aus α-Fluoracrylsäureestern können hochmolekulare, nichtkristalline Polymere hergestellt werden, die transparent sind und Erweichungstemperaturen über 100°C aufweisen.

### Beispiel 1

195 g Chlormalonsäurediethylester wurden in 500 ml Acetonitril gelöst und unter Feuchtigkeitsausschluß mit 320 g des Additionsproduktes von 3 Molen Fluorwasserstoff an 1 Mol Triethylamin versetzt. Dann wurden 100 g Triethylamin zugegeben und bei 80°C Innentemperatur bis zum vollständigen Umsatz gerührt. Anschließend wurde das Lösungsmittel abdestilliert und der Rückstand auf Wasser gegossen. Der erhaltene Fluormalonsäurediethylester der Formel (Ia) wurde mit Methylenchlorid extrahiert, mit gesättigter wäßriger Natriumhydrogencarbonat-Lösung gewaschen, mit Magnesiumsulfat getrocknet und im Hochvakuum fraktioniert. Die Ausbeute betrug 147 g = 83 % der Theorie.

### Beispiel 2

165 g Chloracetessigester wurden in 500 ml Acetonitril gelöst und unter Feuchtigkeitsausschluß mit 320 g des Anlagerungsproduktes von 3 Molen Fluorwasserstoff an 1 Mol Triethylamin versetzt. Dann wurden 100 g Triethylamin zugegeben und bei 80°C Innentemperatur bis zum vollständigen Umsatz gerührt. Anschließend wurde das Lösungsmittel abdestilliert, der Rückstand auf Wasser gegossen, der erhaltene Fluoracetessigester der Formel (Ib) mit Methylenchlorid extrahiert, mit gesättigter wäßriger Natriumhydrogencarbonat-Lösung gewaschen, mit Magnesiumsulfat getrocknet und im Hochvakuum fraktioniert. Die Ausbeute betrug 96 g = 65 % der Theorie.

### Beispiel 3

243 g 2,2-Dibrommalonsäurediethylester wurden mit 480 g des Additionsproduktes von 3 Molen Fluorwasserstoff an 1 Mol Triethylamin versetzt, dann 150 g Triethylamin zugegeben und bei 70°C Innentemperatur gerührt, bis laut GC alles umgesetzt war. Anschließend wurde abgekühlt und das Reaktionsgemisch auf Wasser gegossen. Das Produkt der Formel (Ic) wurde mit Methylenchlorid extrahiert, mit gesättigter wäßriger Natriumhydrogencarbonat-Lösung gewaschen, mit Magnesiumsulfat getrocknet und im Vakuum destilliert.
Man erhielt 108 g (= 72 % der Theorie) 2,2-Difluormalonsäurediethylester mit einem Siedepunkt von 78-80°C bei 10 mbar.

### Beispiel 4

134,6 g 3-Chlorpentan-2,4-dion wurden in 500 ml Acetonitril gelöst und unter Feuchtigkeitsausschluß mit 320 g des Additionsproduktes von 3 Molen Fluorwasserstoff an 1 Mol Triethylamin versetzt. Dann gab man 100 g Triethylamin zu und rührte bei 80°C Innentemperatur bis zum vollständigen Umsatz. Anschließend destillierte man 400 ml Lösungsmittel ab und goß den Rückstand auf Wasser. Das Produkt wurde mit Methylenchlorid extrahiert, mit gesättigter wäßriger Natriumhydrogencarbonat-Lösung gewaschen, mit Magnesiumsulfat getrocknet und im Vakuum fraktioniert. Man erhielt 38 g (= 32 % der Theorie) 3-Fluorpentan-2,4-dion (Formel (Id)) mit einem Siedepunkt von 88°C bei 30 mbar.

### Beispiel 5

25 g 2-Chlor-acetessigsäure-ortho-chloranilid wurden in 75 ml Acetonitril gelöst und unter Feuchtigkeitsausschluß mit 32 g des Additionsproduktes von 3 Molen Fluorwasserstoff an 1 Mol Triethylamin versetzt. Dann gab man 10 g Triethylamin zu und rührte bei 80°C Innentemperatur bis zum vollständigen Umsatz. Anschließend destillierte man 60 ml Lösungsmittel ab und goß den Rückstand auf Wasser, Das Produkt wurde mit Methylenchlorid extrahiert, mit gesättigter wäßriger Natriumhydrogencarbonat-Lösung gewaschen, mit Magnesiumsulfat getrocknet und einrotiert. Zur Reinigung wurde mit Methylenchlorid als Laufmittel über Kieselgel chromatographiert. Man erhielt 15,8 g ( = 69 % der Theorie) 2-Fluor-acetessigsäure-ortho-chloranilid der Formel (Ie).

## Patentansprüche

1. Verfahren zur Herstellung von α-Fluor-β-dicarbonylverbindungen der Formel (I) 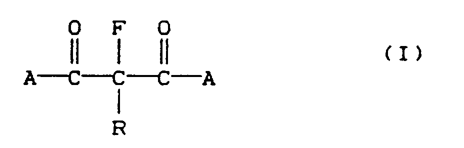 in der
die beiden Reste A gleich oder verschieden sein können und jeweils für Alkyl, Aryl, Alkoxy, Aryloxy oder eine Aminogruppe und
R für Wasserstoff, Fluor, Alkyl oder Aryl stehen,
dadurch gekennzeichnet, daß man eine Dicarbonylverbindung der Formel (II) 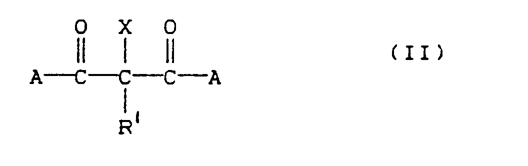 in der
X für Chlor, Brom oder Iod steht,
A die bei Formel (I) angegebene Bedeutung hat und
R' die bei Formel (I) für R angegebene Bedeutung hat und zusätzlich noch für Chlor, Brom oder Iod stehen kann,
bei Temperaturen von 20°C bis 100°C mit einem Anlagerungsprodukt von Fluorwasserstoff an ein Trialkylamin umsetzt.

2. Verfahren nach Anspruch 1, dadurch gekennzeichnet, daß in den Formeln (I) und (II) A für geradkettiges oder verzweigtes, unsubstituiertes oder substituiertes Alkyl, unsubstituiertes oder substituiertes Aryl, geradkettiges oder verzweigtes, unsubstituiertes oder substituiertes Alkoxy, unsubstituiertes oder substituiertes Aryloxy oder eine unsubstituierte oder substituierte Aminogruppe der Formeln (III) bis (V) stehen
NH₂ , (III)
NHR¹ (IV)
und
NR²R³ (V)
in denen
R¹, R² und R³ Alkyl oder Aryl bedeuten und R² und R³ gleich oder verschieden sein können.

3. Verfahren nach Ansprüchen 1 und 2, dadurch gekennzeichnet, daß A in der Bedeutung von Alkyl und Alkoxy 1 bis 6 C-Atome enthält oder Phenyl bedeutet.

4. Verfahren nach Ansprüchen 1 bis 3, dadurch gekennzeichnet, daß in den Formeln (I) und (II) R und R' für Wasserstoff, geradkettiges oder verzweigtes, unsubstituiertes oder substituiertes C₁-C₁₂-Alkyl oder unsubstituiertes oder substituiertes Phenyl stehen und R' zusätzlich für Chlor, Brom oder Iod stehen kann.

5. Verfahren nach Ansprüchen 1 bis 4, dadurch gekennzeichnet, daß in den Formeln (I) und (II) R und R' für Wasserstoff steht.

6. Verfahren nach Ansprüchen 1 bis 5, dadurch gekennzeichnet, daß das Anlagerungsprodukt von Fluorwasserstoff an ein Trialkylamin pro Mol Trialkylamin 1 bis 2,8 Mole Fluorwasserstoff enthält.

7. Verfahren nach Ansprüchen 1 bis 6, dadurch gekennzeichnet, daß es sich bei dem Trialkylamin um Triethylamin handelt.

8. Verfahren nach Ansprüchen 1 bis 7, dadurch gekennzeichnet, daß man bezogen auf Dicarbonylverbindungen der Formel (II) von 1 bis 4 Molen des Anlagerungsproduktes von Fluorwasserstoff an Trialkylamin einsetzt.

9. Verfahren nach Ansprüchen 1 bis 8, dadurch gekennzeichnet, daß man es bei Temperaturen im Bereich 50 bis 90°C durchführt.

10. Verfahren nach Ansprüchen 1 bis 9, dadurch gekennzeichnet, daß man das nach der Reaktion vorliegende Gemisch aufarbeitet, indem man zunächst evtl. vorhandenes Lösungsmittel im Vakuum abzieht, dann das Gemisch auf Wasser austrägt, mit einem mit Wasser nicht mischbaren organischen Lösungsmittel extrahiert und abschließend die hergestellte α-Fluor-β-dicarbonylverbindung der Formel (I) durch Fraktionierung der organischen Phase isoliert.

## Claims

1. Process for the preparation of α-fluoro-β-dicarbonyl compounds of formula (I): 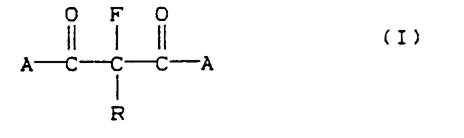 in which
the two radicals A can be identical or different and are each alkyl, aryl, alkoxy, aryloxy or an amino group and
R is hydrogen, fluorine, alkyl or aryl,
characterised in that a dicarbonyl compound of formula (II): 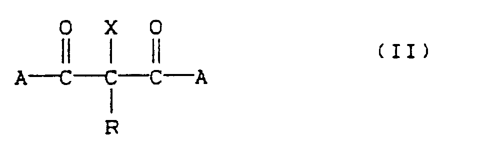 in which
X is chlorine, bromine or iodine,
A is as defined for formula (I) and
R' is as defined for R in formula (I) and can additionally be chlorine, bromine or iodine,
is reacted at temperatures of 20°C to 100°C with an addition product of hydrogen fluoride and a trialkylamine.

2. Process according to Claim 1, characterised in that, in formulae (I) and (II), A is linear or branched, unsubstituted or substituted alkyl, unsubstituted or substituted aryl, linear or branched, unsubstituted or substituted alkoxy, unsubstituted or substituted aryloxy or an unsubstituted or substituted amino group of formulae (III) to (V):
NH₂, (III)
NHR¹ (IV)
and
NR²R³ (V),
in which
R¹, R² and R³ are alkyl or aryl and R² and R³ can be identical or different.

3. Process according to Claims 1 and 2, characterised in that A as alkyl and alkoxy contains 1 to 6 C atoms or A is phenyl.

4. Process according to Claims 1 to 3, characterised in that, in formulae (I) and (II), R and R' are hydrogen, linear or branched, unsubstituted or substituted C₁-C₁₂-alkyl or unsubstituted or substituted phenyl, and R' can additionally be chlorine, bromine or iodine.

5. Process according to Claims 1 to 4, characterised in that, in formulae (I) and (II), R and R' are hydrogen.

6. Process according to Claims 1 to 5, characterised in that the addition product of hydrogen fluoride and a trialkylamine contains 1 to 2.8 mol of hydrogen fluoride per mole of trialkylamine.

7. Process according to Claims 1 to 6, characterised in that the trialkylamine is triethylamine.

8. Process according to Claims 1 to 7, characterised in that from 1 to 4 mol of the addition product of hydrogen fluoride and trialkylamine are used, based on dicarbonyl compounds of formula (II).

9. Process according to Claims 1 to 8, characterised in that it is carried out at temperatures in the range 50 to 90°C.

10. Process according to Claims 1 to 9, characterised in that the mixture obtained after the reaction is worked up by a procedure in which any solvent present is first stripped off under vacuum, the mixture is then discharged into water and extracted with a water-immiscible organic solvent, and the α-fluoro-β-dicarbonyl compound of formula (I) prepared is finally isolated by fractionation of the organic phase.

## Revendications

1. Procédé de préparation de dérivés α-fluoro-β-dicarbonylés de formule I 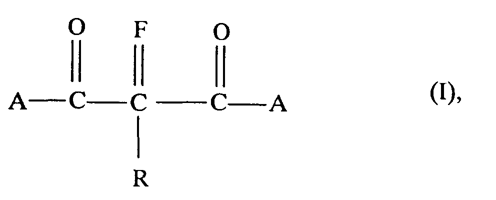 dans laquelle
les deux symboles A, ayant des significations identiques ou différentes, représentent chacun un groupe alkyle, aryle, alcoxy, aryloxy ou un groupe amino et
R représente l'hydrogène, le fluor, un groupe alkyle ou aryle,
caractérisé en ce que l'on fait réagir un dérivé dicarbonylé de formule II 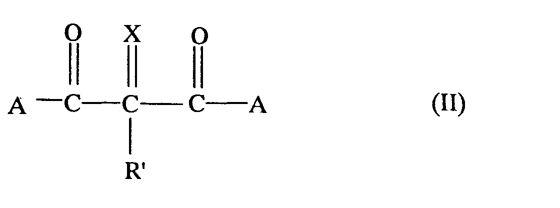 dans laquelle
X représente le chlore, le brome ou l'iode,
A a les significations indiquées en référence à la formule I et
R' a les significations indiquées pour R en référence à la formule I mais peut en outre représenter le chlore, le brome ou l'iode,
à des températures de 20 à 100°C, avec un produit d'addition du fluorure d'hydrogène sur une trialkylamine.

2. Procédé selon la revendication 1, caractérisé en ce que, dans les formules I et II, A représente un groupe alkyle à chaîne droite ou ramifiée, substitué ou non, un groupe aryle substitué ou non, un groupe alcoxy à chaîne droite ou ramifiée, substitué ou non, un groupe aryloxy substitué ou non ou un groupe amino substitué ou non, répondant à l'une des formules III à V
NH₂, (III)
NHR¹ (IV)
et
NR²R³ (V)
dans lesquelles
R¹, R² et R³ représentent des groupes alkyles ou aryles et R² et R³ peuvent être identiques ou différents.

3. Procédé selon les revendications 1 et 2, caractérisé en ce que A représente un groupe alkyle ou un groupe alcoxy en C₁-C₆ ou un groupe phényle.

4. Procédé selon les revendications 1 à 3, caractérisé en que, dans les formules I et II, R et R' représentent l'hydrogène, des groupes alkyles à chaîne droite ou ramifiée, substitués ou non, en C₁-C₁₂, ou des groupes phényles substitués ou non, R' pouvant en outre représenter le chlore, le brome ou l'iode.

5. Procédé selon les revendications 1 à 4, caractérisé en ce que, dans les formules I et II, R et R' représentent l'hydrogène.

6. Procédé selon les revendications 1 à 5, caractérisé en ce que le produit d'addition du fluorure d'hydrogène sur une trialkylamine contient 1 à 2,8 mol de fluorure d'hydrogène par mole de la trialkylamine.

7. Procédé selon les revendications 1 à 6, caractérisé en ce que la trialkylamine est la triéthylamine.

8. Procédé selon les revendications 1 à 7, caractérisé en ce que, pour une mole d'un dérivé dicarbonylé de formule II, on met en oeuvre 1 à 4 mol du produit d'addition du fluorure d'hydrogène sur une trialkylamine.

9. Procédé selon les revendications 1 à 8, caractérisé en ce que l'on opère à des températures dans l'intervalle de 50 à 90°C.

10. Procédé selon les revendications 1 à 9, caractérisé en ce que pour isoler le produit de réaction, on élimine d'abord le solvant éventuel par distillation sous vide, on coule le mélange dans l'eau, on extrait par un solvant organique non miscible à l'eau et finalement on isole le dérivé α-fluoro-β-dicarbonylé de formule I par fractionnement de la phase organique.
